# EUROPÄISCHE PATENTSCHRIFT

(11) **EP 3 037 069 B1**
(45) Veröffentlichungstag und Bekanntmachung des Hinweises auf die Patenterteilung: **18.09.2019**
(21) Anmeldenummer: 15201833.9
(22) Anmeldetag: 22.12.2015
(51) Int. Cl.: A61F 2/46, A61F 2/34, A61F 2/36

(54) **MEDIZINISCHES KRAFTMESSSYSTEM**
MEDICAL FORCE MEASUREMENT SYSTEM
SYSTEME MEDICAL DE MESURE DE FORCE

(30) Priorität: 22.12.2014 DE 102014119348
(43) Veröffentlichungstag der Anmeldung: 29.06.2016
(73) Patentinhaber: AESCULAP AG, 78532 Tuttlingen (DE)
(72) Erfinder: Bader, Uwe, 78532 Tuttlingen (DE); Sungu, Mevlüt, 8200 Schaffhausen (CH)
(74) Vertreter: Hoeger, Stellrecht & Partner Patentanwälte mbB

(56) Entgegenhaltungen:
- WO-A1-2013/170573
- WO-A1-2014/071193
- US-A1- 2007 005 145

## Beschreibung

Die vorliegende Erfindung betrifft ein medizinisches Kraftmesssystem zum Messen einer in einer Gelenkprothese umfassend ein Kugelgelenk oder einer Teilkomponente derselben zwischen zwei gelenkig miteinander verbundenen Prothesenteilen wirkenden Kraft, welches Kraftmesssystem ein Messinstrument umfasst, welches Messinstrument ein Probegelenkelement mit einer Probegelenkfläche umfasst, welches Probegelenkelement korrespondierend zu einem ersten, einen Teil des Kugelgelenks bildenden und eine korrespondierend zur Probegelenkfläche ausgebildete Gelenkfläche aufweisenden Gelenkelement ausgebildet und mit diesem in Eingriff bringbar ist zum Ausbilden eines Probekugelgelenks.

Medizinische Kraftmesssysteme der eingangs beschriebenen Art zum Messen von Kräften sind bislang nicht bekannt. Bekannt sind jedoch Probeimplantatsysteme, wie sie insbesondere in der DE 10 2008 030 261 A1 beschrieben sind. Allerdings ermöglichen es solche Probeimplantatsysteme nicht, einen Einfluss von das künstliche Gelenk umgebenden Weichteilen auf dessen Stabilität zu bestimmen. Dies kann insbesondere wichtig sein, um eine Luxationsneigung des künstlichen Gelenks zu ermitteln und gegebenenfalls zu verringern.

Aus der US 2007/0005145 A1 ist ein intraoperatives Gelenkkraftmesssystem bekannt. Die WO 2014/071193 A1 offenbart Systeme und Verfahren zum Messen orthopädischer Parameter bei Gelenkimplantationen. Die WO 2013/ 170573 A1 betrifft Verfahren und Systeme zum Ermitteln von Stellungen des Acetabulums und des Femurkopfes in Echtzeit während einer Hüftgelenkimplantation.

Es ist daher eine Aufgabe der vorliegenden Erfindung, ein Messsystem der eingangs beschriebenen Art so weiter zu bilden, dass eine Implantation der Gelenkprothese verbessert wird.

Diese Aufgabe wird bei einem medizinischen Kraftmesssystem der eingangs beschriebenen Art erfindungsgemäß dadurch gelöst, dass das Messinstrument eine an dem Probegelenkelement angeordnete Anzeigeeinrichtung umfasst zum Anzeigen einer auf die Probegelenkfläche wirkenden Kraft oder einer Teilkomponente der auf die Probegelenkfläche wirkenden Kraft.

Mit einem solchen medizinischen Kraftmesssystem ist es für einen Arzt, der die Gelenkprothese implantiert, insbesondere möglich, ein sogenanntes schlechtes "Balancing" der Weichteile zu erkennen. Selbst wenn eine Gelenkgeometrie auf Basis einer präoperativen Planung wieder wie ursprünglich rekonstruiert wird, kann es trotzdem aufgrund des gewählten Zugangs zum Operationssitus oder einer veränderten Antetorsion zu einem Ungleichgewicht der auf die Gelenkprothese wirkenden Gelenkkräfte kommen. Das Kraftmesssystem ermöglicht es insbesondere, dem Arzt intraoperativ eine Rückmeldung über eine resultierende, auf die Probegelenkfläche wirkende Kraft zu geben. Dies ist insbesondere deshalb vorteilhaft, weil das "Balancing" der Weichteile einen Einfluss auf die Gelenkstabilität hat. Wirkt die Kraft in Form eines Kraftvektors als Summe aller auf das Gelenk wirkenden Kräfte von insbesondere angreifenden Muskeln ungünstig, so hat dies einen Einfluss auf eine Luxationsneigung. Beispielsweise kann sich dies bei einem Hüftgelenk auf einen Langzeiterfolg des Implantats insbesondere auf Seiten der Gelenkpfanne auswirken. Postoperativ kann ein Kräfteungleichgewicht von auf das Hüftgelenk wirkenden Kräften umliegender Weichteile kommen, und zwar insbesondere durch das Trennen, Verletzen oder Ablösen von Muskelpartien oder aber auch durch veränderte geometrische Abmaße im Gelenk selbst. Postoperativ auftretende Hüftschmerzen können beispielsweise Folge einer fehlenden Osseointegration des Implantats sein oder eben ihre Ursache in dem beschriebenen Kräfteungleichgewicht haben, welches die auftretenden Kräfte in den einzelnen Muskeln im Vergleich zum Zustand vor dem Eingriff verändert, was eine unphysiologische Schmerzsituation im Gelenk bewirken kann. Insbesondere dann, wenn die Schmerzen auch längere Zeit nach dem Eingriff anhalten, muss in einer erneuten Operation versucht werden, die entsprechenden Muskeln durch eine teilweise Trennung zu entlasten. Um eine solche Revisionsoperation aufgrund eines Kräfteungleichgewichts möglichst zu vermeiden, ermöglicht es das medizinische Kraftmesssystem dem Operateur, bereits intraoperativ das Ungleichgewicht zu erkennen und erforderliche Gegenmaßnahmen zu ergreifen.

Günstig ist es, wenn das Messinstrument eine Messinstrumentenkopplungseinrichtung umfasst zum temporären, kraft- und/oder formschlüssigen Koppeln mit einer Kopplungseinrichtung eines Prothesenteils der Gelenkprothese oder einem medizinischen Instrument. Die Messinstrumentenkopplungseinrichtung ermöglicht es insbesondere, das Messinstrument temporär mit einem Prothesenteil, beispielsweise einem Hüftschafft oder einer Hüftpfanne eines künstlichen Hüftgelenks, oder einem medizinischen Instrument zu koppeln. Das Messinstrument kann dann mit dem Prothesenteil oder einem medizinischen Instrument mit seinem Probegelenkelement zusammen mit einem bereits implantierten Gelenkelement der zu implantierenden Gelenkprothese temporär gekoppelt werden zur Ausbildung eines Probekugelgelenks.

Vorteilhafterweise umfasst das medizinische Kraftmesssystem ein medizinisches Instrument, welches das Messinstrument umfasst oder mit dem Messinstrument koppelbar ist. Insbesondere kann das Messinstrument bereits am medizinischen Instrument angeordnet sein. Alternativ kann es mit dem Messinstrument auch temporär koppelbar sein. Dies ermöglicht es insbesondere, während der Implantation das Messinstrument wahlweise mit dem Instrument zu koppeln und gegebenenfalls mit einem Prothesenteil der Gelenkprothese.

Günstig ist es, wenn das medizinische Instrument in Form einer Raspel oder eines Raspelschafts ausgebildet ist. Beispielsweise kann mit einer Raspel eine Knochenkavität an einem Femur präpariert werden, um einen Prothesenschaft einer Hüftgelenkendoprothese einzusetzen. So kann beispielsweise eine Kraft auf die Probegelenkfläche des mit der Raspel oder dem Raspelschaft gekoppelten Messinstruments bestimmt werden, wenn die Raspel oder der Raspelschaft in die Knochenkavität eingebracht ist.

Um eine einfache Verbindung des medizinischen Instruments mit dem Messinstrument zu ermöglichen, ist es günstig, wenn das medizinische Instrument eine Instrumentenkopplungseinrichtung umfasst, welche mit der Messinstrumentenkopplungseinrichtung kraft- und/oder formschlüssig in Eingriff bringbar ist.

Auf besonders einfache Weise lassen sich das medizinische Instrument und das Messinstrument miteinander koppeln, wenn die Instrumentenkopplungseinrichtung oder die Messinstrumentenkopplungseinrichtung in Form eines Kopplungsvorsprungs ausgebildet ist und wenn die jeweils andere Kopplungseinrichtung in Form einer zum Kopplungsvorsprung korrespondierenden Kopplungsaufnahme ausgebildet ist. Beispielsweise kann das Instrument einen Kopplungsvorsprung oder eine Kopplungsaufnahme aufweisen, ebenso kann das Messinstrument einen Kopplungsvorsprung oder eine Kopplungsaufnahme aufweisen.

Gemäß einer weiteren bevorzugten Ausführungsform der Erfindung kann vorgesehen sein, dass der Kopplungsvorsprung am Prothesenteil oder am Instrument angeordnet oder ausgebildet ist und die Kopplungsaufnahme am Messinstrument angeordnet oder ausgebildet ist oder dass die Kopplungsaufnahme am Prothesenteil oder am Instrument angeordnet oder ausgebildet ist und der Kopplungsvorsprung am Messinstrument angeordnet oder ausgebildet ist. Die beschriebenen Weiterbildungen ermöglichen es insbesondere, das Messinstrument in Form oder als Teil eines Probegelenkkopfes oder aber auch als Teil einer Probegelenkpfanne oder eines Probegelenkpfanneneinsatzes einer Hüftgelenkendoprothese auszubilden.

Vorzugsweise umfasst das medizinische Kraftmesssystem ein Prothesenteil der Gelenkprothese, welches mit dem Messinstrument temporär koppelbar ist. Beispielsweise kann es sich dabei um das dauerhaft zu implantierende Prothesenteil oder um ein Probeprothesenteil handeln, welches nur temporär während des chirurgischen Eingriffs zum Einsatz kommt.

Insbesondere für die Implantation einer Hüftgelenkendoprothese ist es vorteilhaft, wenn das Prothesenteil in Form eines in eine Knochenkavität implantierbaren Prothesenschafts oder in Form einer Gelenkpfanne oder eines Gelenkpfanneneinsatzes einer Gelenkpfanne ausgebildet ist.

Günstig ist es, wenn das Probegelenkelement in Form eines Probegelenkkopfes oder in Form eines Pfanneneinsatzes einer Hüftgelenkprothese ausgebildet ist. Dies ermöglicht es einem Operateur insbesondere, das Probegelenkelement in Form eines Probegelenkkopfes mit einer bereits implantierten Gelenkpfanne temporär zu koppeln, um auf das Gelenk wirkende Weichteilkräfte zu bestimmen. Alternativ kann das Probegelenkelement in Form eines Pfanneneinsatzes einer Hüftgelenkprothese auch mit einem Gelenkkopf eines bereits in eine Knochenkavität implantierten Schafts temporär gekoppelt werden, um eine Resultierende der wirkenden Weichteilkräfte zu bestimmen.

Besonders vielseitig anwenden lässt sich das Kraftmesssystem, wenn das Messinstrument ein Kopplungselement umfasst und wenn das Kopplungselement die Messinstrumentenkopplungseinrichtung umfasst. Insbesondere lässt sich so das Messinstrument zweiteilig oder mehrteilig ausbilden, wenn es das Probegelenkelement und das Kopplungselement umfasst und gegebenenfalls noch weitere Teile oder Elemente.

Um auf einfache Weise Kräfte bestimmen zu können, die zwischen dem Probegelenkelement und dem Kopplungselement wirken, ist es günstig, wenn das Probegelenkelement und das Kopplungselement relativ zueinander beweglich angeordnet oder aneinander gelagert oder relativ zueinander beweglich ausgebildet sind. Insbesondere lässt sich so mechanisch eine wirkende Kraft messen, die auf die Probegelenkfläche wirkt, um das Probegelenkelement relativ zum Kopplungselement zu bewegen.

Vorzugsweise sind das Probegelenkelement und das Kopplungselement relativ zueinander verschiebbar und/oder verdrehbar angeordnet oder ausgebildet. Insbesondere können das Probegelenkelement und das Kopplungselement relativ zueinander in allen Raumrichtungen beweglich angeordnet oder ausgebildet sein. Auch ist es denkbar, einen, zwei oder drei Bewegungsfreiheitsgrade des Probegelenkelements und des Kopplungselements ganz oder teilweise einzuschränken.

Auf besonders einfache Weise lässt sich das Messinstrument mit einem Prothesenteil oder einem medizinischen Instrument temporär koppeln, wenn die Instrumentenkopplungseinrichtung ein Klemmelement umfasst, welches einerseits mit dem Prothesenteil oder dem medizinischen Instrument und andererseits mit dem Kopplungselement kraft- und/oder formschlüssig in Eingriff bringbar ist.

Besonders kompakt ausbilden lässt sich das Kraftmesssystem, wenn das Klemmelement in Form eines Klemmrings ausgebildet ist, welcher in einer Klemmstellung in eine Ringnut am Prothesenteil oder am Instrument einerseits und/oder in eine Ringnut am Kopplungselement eingreift. Beispielsweise kann so das Messinstrument auf einen Hals des medizinischen Instruments, beispielsweise einen Hals einer Raspel, oder auf einen Hals eines Prothesenschafts aufgebracht und dort klemmend gehalten werden.

Um das Verbinden des Messinstruments mit einem Prothesenteil oder einem Instrument zu erleichtern, ist es günstig, wenn das Klemmelement elastisch und/oder flexibel ausgebildet ist.

Um einem Operateur eine wirkende Kraft intraoperativ anzeigen zu können, ist es vorteilhaft, wenn die Anzeigeeinrichtung mindestens ein Anzeigeelement und mindestens ein Referenzelement umfasst und wenn das mindestens eine Anzeigeelement und das mindestens eine Referenzelement relativ zueinander beweglich angeordnet oder ausgebildet sind. Eine solche Anzeigeeinrichtung ermöglicht insbesondere, eine Bewegung beziehungsweise Auslenkung des mindestens einen Anzeigeelements und des mindestens einen Referenzelements relativ zueinander einem Operateur direkt sichtbar oder erkennbar zu machen. Insbesondere kann die Anzeigeeinrichtung rein mechanisch ausgebildet sein, sodass keine weiteren Elemente zum Anzeigen einer wirkenden Kraft oder einer Teilkomponente derselben erforderlich sind.

Ferner kann es günstig sein, wenn die Anzeigeeinrichtung eine Mehrzahl von Anzeigeelementen umfasst, welche in Folge einer Krafteinwirkung auf das Probegelenkelement und/oder das Kopplungselement aus einer Grundstellung auslenkbar und/oder temporär verformbar sind. Insbesondere kann die Mehrzahl von Anzeigeelementen derart angeordnet sein, dass eine Auslenkung oder Verformung eines oder mehrerer der Anzeigeelemente direkt einen Rückschluss zulässt, aus welcher Richtung und gegebenenfalls mit welcher Größe eine Kraft auf das Probegelenkelement und insbesondere dessen Probegelenkfläche wirkt.

Auf besonders einfache Weise ausbilden lässt sich das Messinstrument, wenn die Mehrzahl von Anzeigeelementen in Form von gekrümmten Lamellen ausgebildet sind, welche vom Kopplungselement abstehen und vom Probegelenkelement weg weisen. Insbesondere können die Lamellen derart angeordnet sein, dass bei einer Relativbewegung zwischen dem Kopplungselement und dem Probegelenkelement eine oder mehrere der Lamellen ausgelenkt und/oder verformt werden.

Um auf einfache Weise eine Orientierung der wirkenden Kraft anzeigen zu können, ist es vorteilhaft, wenn die Mehrzahl von Anzeigeelementen die Kopplungsaufnahme in Umfangsrichtung umgebend angeordnet oder ausgebildet ist. Beispielsweise kann so bei einer Auslenkung oder Verformung eines oder mehrerer der Anzeigeelemente direkt eine Richtung der wirkenden Kraft oder zumindest einer Teilkomponente derselben angezeigt werden.

Günstig ist es, wenn das Kopplungselement eine Kopplungselementlängsachse definiert und wenn die Mehrzahl von Anzeigeelementen die Kopplungselementlängsachse umgebend angeordnet ist. Insbesondere kann die Mehrzahl von Anzeigeelementen gleichmäßig in Umfangsrichtung die Kopplungselementlängsachse umgebend angeordnet oder ausgebildet sein. So kann insbesondere eine Kraft oder eine Kraftkomponente, die beispielsweise zwischen dem Probegelenkelement und dem Kopplungselement wirkt, bezogen auf die Kopplungselementlängsachse angezeigt werden.

Um die Handhabung des medizinischen Kraftmesssystems zu verbessern, ist es vorteilhaft, wenn das Kopplungselement und das Probegelenkelement kraft- und/oder formschlüssig miteinander gekoppelt sind. Auf diese Weise kann insbesondere verhindert werden, dass sich das Kopplungselement und das Probegelenkelement nicht unbeabsichtigt voneinander lösen können.

Auf besonders einfache Weise lassen sich das Kopplungselement und das Probegelenkelement miteinander verbinden, wenn sie klemmend oder rastend miteinander gekoppelt sind. Hierzu können Klemmelemente vorgesehen sein oder aber auch zusammenwirkende und/oder ineinandergreifende Teile des Kopplungselements und des Probegelenkelements. Insbesondere kann eine Verbindung von Kopplungselement und Probegelenkelement auch mit einer Presspassung hergestellt werden. Optional oder alternativ können auch Rastelemente am Kopplungselement und am Probegelenkelement angeordnet oder ausgebildet sein, die in einer Verbindungsstellung kraft- und/oder formschlüssig miteinander in Eingriff stehen.

Gemäß einer weiteren bevorzugten Ausführungsform der Erfindung kann vorgesehen sein, dass das mindestens eine Anzeigeelement in Form Ringfläche am Probegelenkelement oder am Kopplungselement ausgebildet ist und dass das mindestens eine Referenzelement in Form eines Rings ausgebildet ist, welcher einen kleineren Durchmesser aufweist als die Ringfläche und diese mindestens teilweise überdeckt. Diese Ausgestaltung ermöglicht insbesondere die Ausbildung einer Anzeigeeinrichtung, welche eine Relativbewegung zwischen dem Ring und der Ringfläche sichtbar macht. Wirkt beispielsweise eine Kraft auf das Probegelenkelement, so kann dieses optional relativ zum Kopplungselement bewegt werden, wobei dann ebenfalls der Ring und die Ringfläche relativ zueinander bewegt werden. Eine Verschiebung einer in einer Grundstellung konzentrischen Ausrichtung zwischen dem Ring und der Ringfläche ermöglicht es einem Operateur, direkt zu erkennen, aus welcher Richtung oder in welche Richtung eine von den Weichteilen auf das Probegelenkelement wirkende Kraft weist.

Auf besonders einfache Weise ausbilden lässt sich das Messinstrument, wenn die Ringfläche in Form einer ebenen, vom Probegelenkelement weg weisenden Endfläche ausgebildet ist, welche eine Ausnehmung des Probegelenkelements umgibt, in welche das Kopplungselement eingreift, und wenn das Kopplungselement einen die Kopplungsaufnahme umgebenden, von einer Kopplungsaufnahmelängsachse radial weg weisenden Ringflansch umfasst, welcher den Ring bildet. So kann auf einfache Weise eine Auslenkung des Probegelenkelements und des Kopplungselements relativ zueinander sichtbar gemacht werden, wenn nämlich die in einer Grundstellung vom Ring teilweise verdeckte Ringfläche, die in der Grundstellung konzentrisch zur Kopplungsaufnahmelängsachse sichtbar ist, eine sichtbar unrunde Form annimmt aufgrund einer Relativbewegung zwischen dem Probegelenkelement und dem Kopplungselement.

Günstig ist es, wenn das Kopplungselement und das Probegelenkelement axial bezogen auf die Kopplungselementlängsachse relativ zueinander festgelegt sind. So kann insbesondere vermieden werden, dass sich das Probegelenkelement und das Kopplungselement parallel zur Kopplungselementlängsachse voneinander in unbeabsichtigter Weise trennen können.

Um eine Beweglichkeit des Messinstruments zu verbessern, ist es vorteilhaft, wenn das Kopplungselement und das Probegelenkelement relativ zueinander um die Kopplungselementlängsachse verdrehbar gekoppelt sind. Optional können sie auch in einer Ebene, die senkrecht oder quer zur Kopplungselementlängsachse verläuft, relativ zueinander verschiebbar angeordnet oder ausgebildet sein.

Vorzugsweise umfassen das Kopplungselement und das Probegelenkelement zusammenwirkende Kopplungsglieder, die formschlüssig oder im Wesentlichen formschlüssig in Eingriff stehen. Beispielsweise kann es sich um zusammenwirkende Vorsprünge und Ausnehmungen handeln, die am Probegelenkelement einerseits oder am Kopplungselement andererseits angeordnet oder ausgebildet sind.

Um insbesondere eine Bewegung in einer Ebene senkrecht oder quer zur Kopplungselementlängsachse zu gestatten, ist es vorteilhaft, wenn die zusammenwirkenden Kopplungsglieder eine Ringnut und einen korrespondierenden Ringflansch umfassen, die einerseits am Kopplungselement und andererseits am Probegelenkelement angeordnet oder ausgebildet sind.

Günstig ist es, wenn ein minimaler Innendurchmesser der Ringnut größer ist als ein maximaler Außendurchmesser des Ringflanschs. So kann auf einfache Weise ein gewünschtes Spiel beziehungsweise eine gewünschte Beweglichkeit des Ringflanschs in der Ringnut vorgegeben werden.

Um einerseits eine sichere Kopplung und andererseits eine möglichst uneingeschränkte Beweglichkeit in gewünschter Weise zu erreichen, ist es vorteilhaft, wenn eine Höhe des Ringflanschs in radialer Richtung größer ist als ein halbe Tiefe der Ringnut in radialer Richtung.

Gemäß einer weiteren bevorzugten Ausführungsform der Erfindung kann vorgesehen sein, dass das Messinstrument eine mit dem Probegelenkelement zusammenwirkende Messeinrichtung umfasst zum Messen einer auf die Probegelenkfläche wirkenden Kraft oder einer Teilkomponente der auf die Probegelenkfläche wirkenden Kraft umfasst, wobei insbesondere die Messeinrichtung mindestens einen Kraftmesssensor umfasst zum Messen einer Größe und/oder Richtung einer auf ihn wirkenden Kraft. Insbesondere können auch zwei, drei oder mehr Kraftmesssensoren vorgesehen sein.

Besonders einfach und kompakt ausbilden lässt sich das Messinstrument, wenn der mindestens eine Kraftmesssensor zwischen dem Probegelenkelement und dem Kopplungselement angeordnet ist. So kann auf einfache Weise eine Relativkraft, die zwischen dem Kopplungselement und dem Probegelenkelement wirkt, gemessen werden.

Um eine Genauigkeit der Messung der zu bestimmenden Kraft zu verbessern, ist es vorteilhaft, wenn eine Mehrzahl von Kraftmesssensoren vorgesehen ist.

Günstigerweise ist der mindestens eine Kraftmesssensor an einem der zusammenwirkenden Kopplungsglieder angeordnet. Dies ermöglicht einen besonders kompakten Aufbau des Messinstruments.

Um den mindestens einen Kraftmesssensor sicher und definiert anordnen zu können, ist es vorteilhaft, wenn mindestens eines der Kopplungsglieder mindestens eine Sensoraufnahme zum Aufnehmen des mindestens einen Kraftmesssensors umfasst. Insbesondere kann die Sensoraufnahme so ausgebildet sein, dass der mindestens eine Kraftmesssensor kraft- und/oder formschlüssig in diese eingreift oder in der Sensoraufnahme in definierter Weise beweglich gehalten ist.

Besonders kompakt ausbilden lässt sich die Messeinrichtung, wenn die mindestens eine Sensoraufnahme am Ringflansch angeordnet oder ausgebildet ist.

Vorzugsweise ist der mindestens eine Kraftmesssensor in Form eines elektronischen Kraftmesssensors ausgebildet zum Erzeugen eines elektrischen Messsignals. Das Messsignal kann so insbesondere beliebig weiter verarbeitet werden, beispielsweise mit einem Computer. Der Kraftmesssensor kann in Form eines digitalen oder analogen Kraftmesssensors ausgebildet sein, welcher ein analoges oder digitales elektrisches Messsignal erzeugen kann.

Gemäß einer weiteren bevorzugten Ausführungsform der Erfindung kann vorgesehen sein, dass der mindestens eine Kraftmesssensor eine Sendeeinrichtung umfasst zum Senden eines Messsignals an eine Empfangseinrichtung einer Signalanzeigeeinrichtung zum Auswerten und/oder Anzeigen des Messsignals. Eine solche Sendeeinrichtung ermöglicht es, das Messsignal an die Empfangseinrichtung der Signalanzeigeeinrichtung zu leiten, um es dort weiter zu verarbeiten und gegebenenfalls anzuzeigen.

Ein Operateur kann ein Kräfteungleichgewicht im Bereich des zu implantierenden Gelenks auf einfache Weise erkennen, wenn die Signalanzeigeeinrichtung einen Bildschirm umfasst. So kann auf dem Bildschirm insbesondere eine Kraft entsprechend ihrer bestimmten Größe und/oder Orientierung dargestellt werden, sodass er zur Herstellung des gewünschten Kräftegleichgewichts erforderliche Maßnahmen ergreifen kann.

Vorteilhaft ist es, wenn die Sendeeinrichtung und/oder die Empfangseinrichtung ausgebildet sind für eine kabelgebundene oder kabellose Übertragung des Messsignals. Insbesondere kann eine kabellose Übertragung über eine Nahfeldkommunikationsschnittstelleneinrichtung über Funk oder Bluetooth oder dergleichen erreicht werden.

Die Herstellung des medizinischen Kraftmesssystems lässt sich weiter vereinfachen, wenn das Probegelenkelement einstückig ausgebildet ist und/oder wenn das Kopplungselement einstückig ausgebildet ist. So lässt sich das Kraftmessinstrument insbesondere nur aus zwei Teilen ausbilden.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Gesamtansicht eines ersten Ausführungsbeispiels eines Kraftmesssystems;
- Figur 2:: eine Teilansicht der Anordnung aus Figur 1 in einer Explosionsdarstellung;
- Figur 3:: eine Schnittansicht längs Linie 3 - 3 in Figur 1;
- Figur 4:: eine vergrößerte Teilansicht der Anordnung aus Figur 1 bei mit auf das Probegelenkelement wirkenden Kraft;
- Figur 5:: eine Schnittansicht längs Linie 5 - 5 in Figur 4;
- Figur 6:: eine schematische perspektivische Ansicht eines zweiten Ausführungsbeispiels eines medizinischen Messsystems;
- Figur 7:: eine Schnittansicht längs Linie 7 - 7 in Figur 6;
- Figur 8:: eine Schnittansicht ähnlich Figur 7, jedoch mit relativ zum Kopplungselement ausgelenktem Probegelenkelement;
- Figur 9:: eine perspektivische, teilweise durchbrochene Gesamtansicht eines dritten Ausführungsbeispiels eines medizinischen Messsystems;
- Figur 10:: eine Teilansicht der Anordnung aus Figur 9 mit abgenommenem Probegelenkelement;
- Figur 11:: eine Schnittansicht längs Linie 11 - 11 in Figur 9; und
- Figur 12:: eine Schnittansicht längs Linie 12 - 12 in Figur 9.

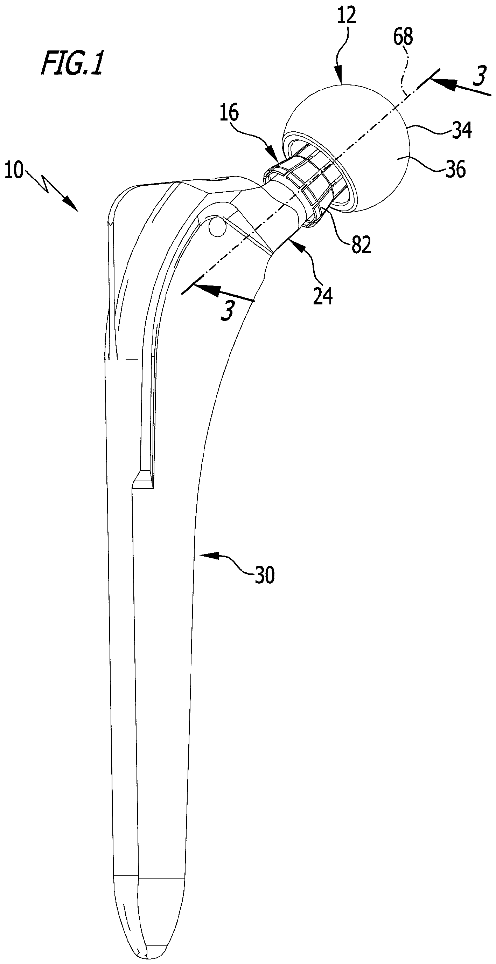

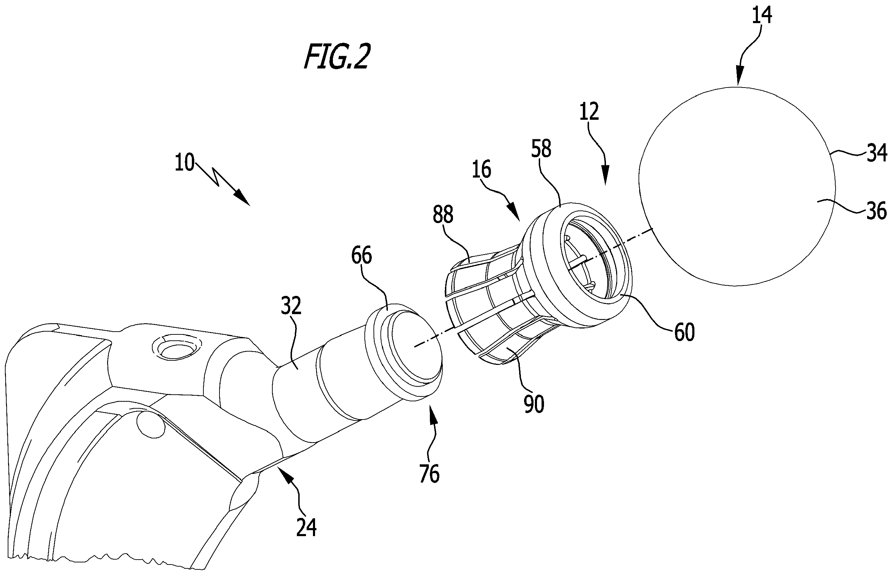

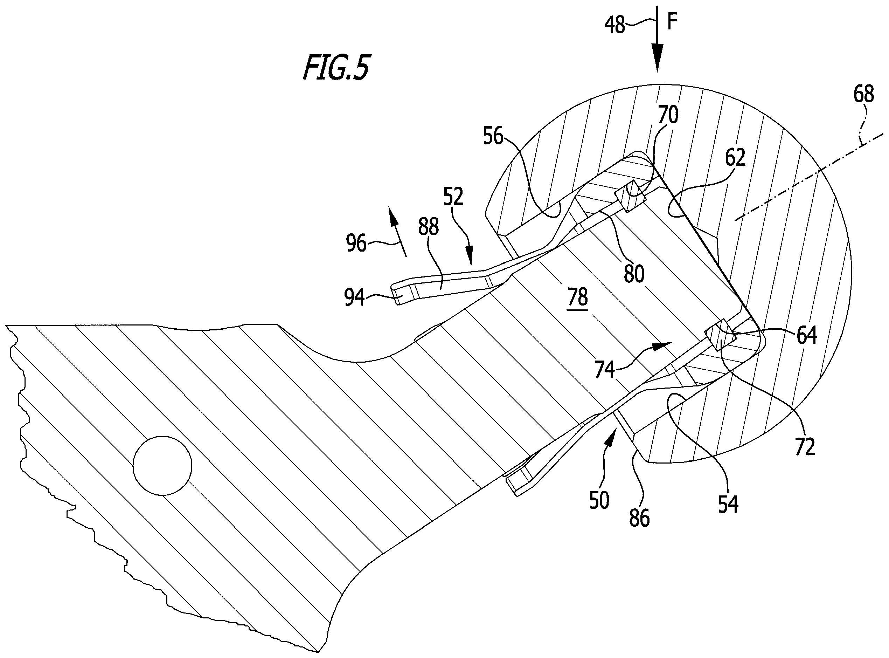

In den Figuren 1 bis 5 ist beispielhaft ein erstes Ausführungsbeispiel eines insgesamt mit dem Bezugszeichnen 10 bezeichneten medizinischen Kraftmesssystems dargestellt. Es umfasst ein Messinstrument 12 mit einem Probegelenkelement 14 und einem Kopplungselement 16.

Das medizinische Kraftmesssystem 10 ist ausgebildet zum Messen einer in einer Gelenkprothese 18 umfassend ein Kugelgelenk oder eine Teilkomponente 22 der Gelenkprothese 18 zwischen zwei gelenkig miteinander verbundenen Prothesenteilen 24 und 26 wirkenden Kraft.

Bei dem in den Figuren 1 bis 5 schematisch dargestellten Ausführungsbeispiel ist die Gelenkprothese 18 in Form einer Hüftgelenkendoprothese 28 ausgebildet mit einem in eine Knochenkavität eines Femurs einsetzbaren Schaft 30, welcher einen Hals 32 an seinem proximalen Ende aufweist, welcher mit dem Messinstrument 12 temporär koppelbar ist. Optional kann die Gelenkprothese 18 auch modular ausgebildet sein, sodass der Schaft 30 und der Hals 32 jeweils in Form separater Prothesenteile ausgebildet sind, die in Abhängigkeit von einer Größe eines Patienten miteinander beliebig koppelbar sind.

Das Probegelenkelement 14 ist in Form eines Probegelenkkopfs 34 mit einer sphärischen Probegelenkfläche 36 ausgebildet. Die Probegelenkfläche 36 ist korrespondierend zu einer Gelenkfläche 38 des Prothesenteils 26 ausgebildet. Der Prothesenteil 26 ist bei dem in den Figuren dargestellten Ausführungsbeispiel in Form einer in einen Beckenknochen implantierbaren Hüftpfanne 40 mit einem kraft- und/oder formschlüssig in dieser festlegbaren Pfanneneinsatz 42 ausgebildet, wobei der Pfanneneinsatz 42 die hohlkugelige Gelenkfläche 38 umfasst.

Das Messinstrument 12 kann optional auch ausgebildet sein zum Koppeln mit einem medizinischen Instrument, beispielsweise in Form einer Raspel oder eines Raspelschafts, der genutzt wird, um die Knochenkavität zum Einbringen des Schafts 30 vorzubereiten. Der Raspelschaft oder die Raspel weisen an ihrem proximalen Ende eine Instrumentenkopplungseinrichtung in Form eines Halses ähnlich dem Hals 32 auf, sodass das Messinstrument 12 auch mit dem medizinischen Instrument temporär koppelbar ist.

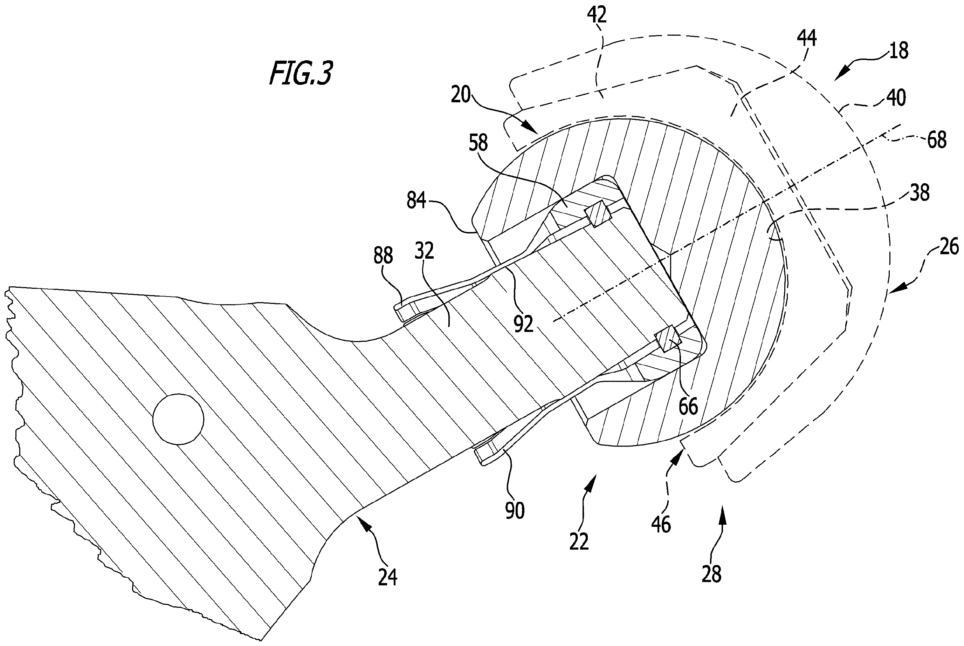

Wird, wie schematisch in Figur 3 dargestellt, das Probegelenkelement 14 mit dem ein Gelenkelement 44 bildenden Pfanneneinsatz 42 in Eingriff gebracht, wird temporär insgesamt ein Probekugelgelenk 46 ausgebildet.

Zum Messen und/oder Anzeigen einer auf die Probegelenkfläche 36 wirkenden Kraft oder einer Teilkomponente der auf die Probegelenkfläche 36 wirkenden Kraft 48 umfasst das Messinstrument 12 eine mit dem Probegelenkelement 14 zusammenwirkende Messeinrichtung 50 und eine Anzeigeeinrichtung 52.

Der Probegelenkkopf 34 weist eine Ausnehmung 54 in Form eines hohlzylindrischen Sacklochs 56 auf. Das Kopplungselement 16 umfasst einen hohlzylindrischen Ring 58, welcher im Presssitz in der Ausnehmung 54 sitzt und an der proximalen Endfläche 60 an einem Boden 62 des Sacklochs 56 anliegt.

Am Hals 32 ist eine umlaufende Ringnut 64 ausgebildet, in welche ein Klemmelement 66 eingesetzt ist. Das Klemmelement 66 greift in eine auf eine Kopplungselementlängsachse 68 hin weisend geöffnete Ringnut 70 ein zum Koppeln des Prothesenteils 24 mit dem Messinstrument 12, nämlich mit dessen Kopplungselement 16. Das Klemmelement 66 ist in Form eines Klemmrings 72 ausgebildet, welcher vorzugsweise elastisch und/oder flexibel ausgebildet ist, um das in Eingriff Bringen sowohl mit dem Prothesenteil 24 als auch mit dem Kopplungselement 16 zu vereinfachen.

Das Messinstrument 12 umfasst eine Messinstrumentenkopplungseinrichtung 74 zum temporären, kraft- und/oder formschlüssigen Koppeln mit einer Kopplungseinrichtung 76 des Prothesenteils 24. Die Kopplungseinrichtung 76 umfasst den Hals 32 und die Ringnut 64 sowie das Klemmelement 66. Die Messinstrumentenkopplungseinrichtung 74 umfasst insbesondere die Ringnut 70.

Wird statt des Schafts 30 ein medizinisches Instrument in Form einer Raspel verwendet, bilden diejenigen Teile der Kopplungseinrichtung 76 die oben erwähnte Instrumentenkopplungseinrichtung.

Alternativ kann das Kopplungselement 16 auch einstückig mit dem medizinischen Instrument, beispielsweise in Form eines Raspelschafts oder einer Raspel, ausgebildet sein.

Die Kopplungseinrichtung 76 umfasst einen Kopplungsvorsprung 78, welcher im Wesentlichen durch den Hals 32 gebildet wird, die Messinstrumentenkopplungseinrichtung 74 umfasst eine Kopplungsaufnahme 80, die im Wesentlichen korrespondierend zum Kopplungsvorsprung 78 ausgebildet ist. Damit ist der Kopplungsvorsprung 78 am Prothesenteil 24 oder alternativ am Instrument angeordnet oder ausgebildet, die Kopplungsaufnahme 80 am Messinstrument 12. Alternativ kann die Kopplungsaufnahme auch am Prothesenteil oder am Instrument angeordnet oder ausgebildet sein, der Kopplungsvorsprung am Messinstrument.

Die Anzeigeeinrichtung 52 umfasst mehrere Anzeigeelemente 82 sowie ein Referenzelement 84 in Form einer in distaler Richtung vom Probegelenkelement 14 weg weisenden, ebenen Endfläche 86. Die Anzeigeelemente 82 und das Referenzelement 84 sind relativ zueinander beweglich ausgebildet.

Die Anzeigeelemente 82 sind in Form von im Wesentlichen von der Kopplungselementlängsachse 68 weg weisend konkav gekrümmten Streifen 90 ausgebildet, welche einen kurzen Anlageabschnitt 92 aufweisen, mit welchem sie am Hals 32 anliegen. Im Wesentlichen erstrecken sich die Streifen 90 parallel zur Kopplungselementlängsachse 68 und sind einstückig mit dem Kopplungselement 16 ausgebildet. Sie erstrecken sich vom Ring 58 weg aus der Ausnehmung 54 heraus.

In einer Grundstellung, wie sie schematisch in den Figuren 1 bis 3 dargestellt ist, umgeben die Lamellen 80 die Kopplungselementlängsachse 68 konzentrisch.

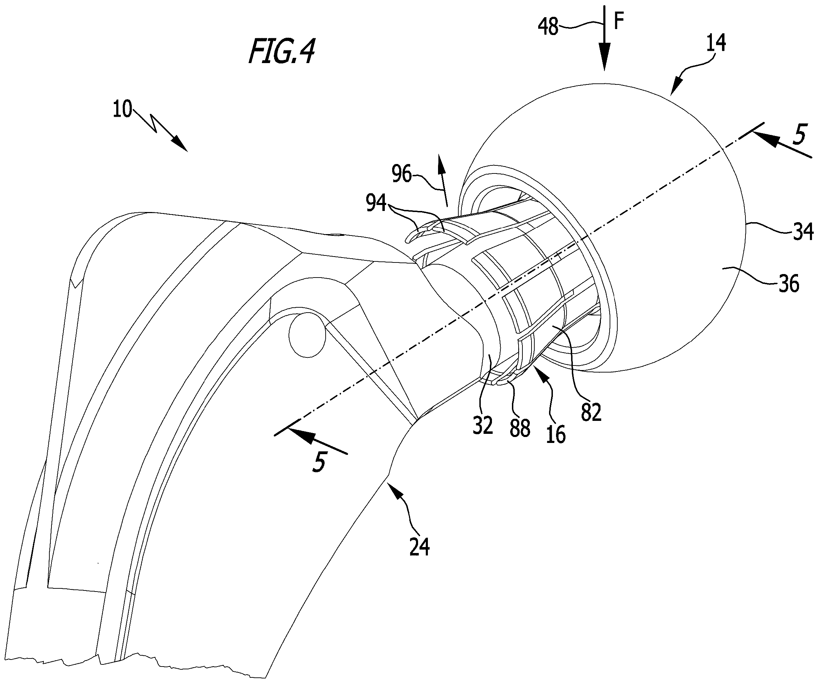

Wird auf die Probegelenkfläche 36 eine Kraft 48 ausgeübt, bewegt sich das Probegelenkelement 14 mit dem Kopplungselement 16 relativ zum Hals 32 derart, dass mindestens eine der Lamellen 88 so verformt wird, dass deren freies Ende 94 in eine Richtung 96 weist, welche im Wesentlichen eine Richtung der Kraft 48 entgegengerichtet ist. Je nach Wirkrichtung der Kraft 48 können auch zwei oder mehr Lamellen 88 in der beschriebenen Weise verformt werden, wie dies beispielhaft in Figur 4 dargestellt ist.

Durch die Auslenkung der Anzeigeelemente 82 kann ein Operateur direkt erkennen, aus welcher Richtung und, in Abhängigkeit einer Größe der Auslenkung der Anzeigeelemente 82 relativ zum Referenzelement 84, gegebenenfalls auch wie groß die Kraft 48 ist.

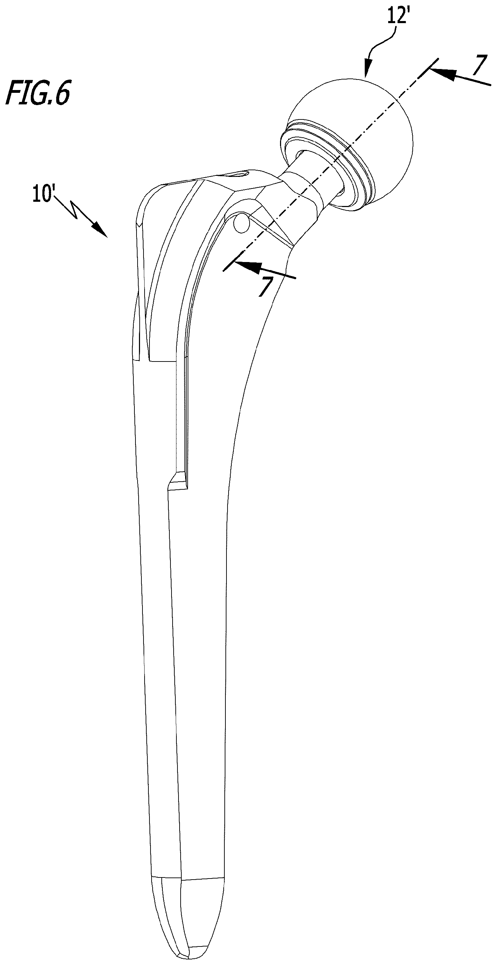

In den Figuren 6 bis 8 ist ein insgesamt mit dem Bezugszeichen 10' bezeichnetes zweites Ausführungsbeispiel eines medizinischen Kraftmesssystems schematisch dargestellt. Es unterscheidet sich im Wesentlichen im Aufbau des Messinstruments 12' vom Messsystem 10. Der Übersichtlichkeit halber sind daher nachfolgend alle Teile und Elemente des Kraftmesssystems 10' mit identischen Bezugszeichen unter Anfügung eines Anstrichs bezeichnet.

Der Prothesenteil 24' unterscheidet sich vom Prothesenteil 24 lediglich dadurch, dass der Hals 32' keine Ringnut aufweist. Er bildet die Kopplungseinrichtung 76' in Form eines Kopplungsvorsprungs 78', welcher in die sacklochförmige Kopplungsaufnahme 80' des hohlzylindrischen Kopplungselements 16' eingreift. Ein proximales Ende des Kopplungselements 60' ist mit einer scheibenförmigen Stirnwand 98' verschlossen. An deren Innenseite 100' schlägt eine in proximaler Richtung weisende ebene Endfläche 102' des Halses 32' an.

Die Kopplungsaufnahme 80' bildet einen Teil der Messinstrumentenkopplungseinrichtung 74'. Der Hals 32' sitzt im Presssitz in der Kopplungsaufnahme 80'.

Ein distales Ende des Kopplungselements 16' wird gebildet durch einen in radialer Richtung von der Kopplungselementlängsachse 68' weg weisenden Ringflansch 104', welcher einen kreisförmigen Ring 106' definiert. Ferner ist am Kopplungselement 16' zwischen der Stirnwand 98' und dem Ringflansch 104' ein weiterer, in radialer Richtung von der Kopplungselementlängsachse 68' weg weisender Ringflansch 108' ausgebildet. Er bildet ein Kopplungsglied 110', welches mit einem weiteren Kopplungsglied 112', welches in Form einer in Richtung auf die Kopplungselementlängsachse 68' hin weisend geöffneten Ringnut 114' ausgebildet ist, in Eingriff steht. Eine Breite 116' des Ringflansches 108' sowie der Ringnut 114' parallel zur Kopplungselementlängsachse 68' ist im Wesentlichen identisch.

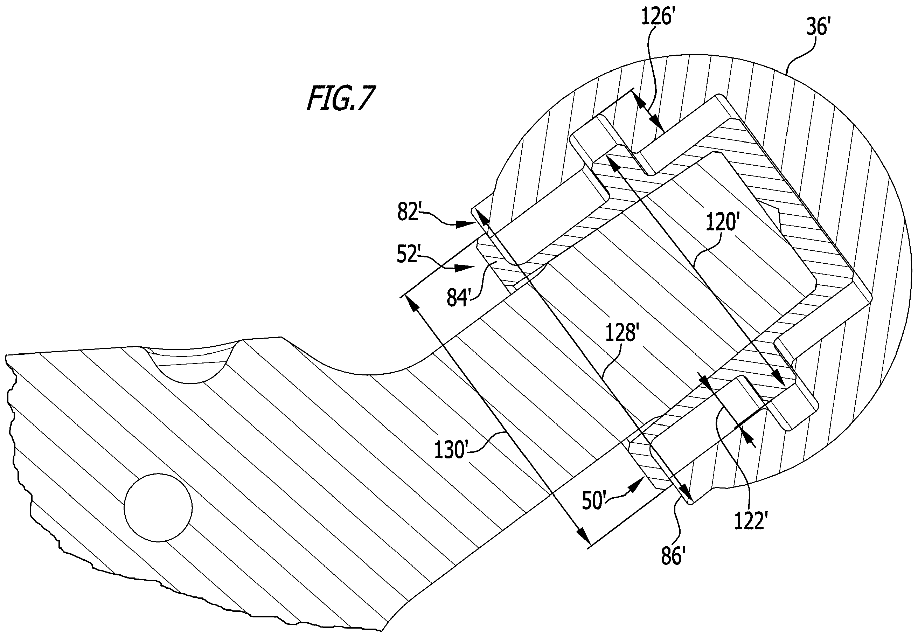

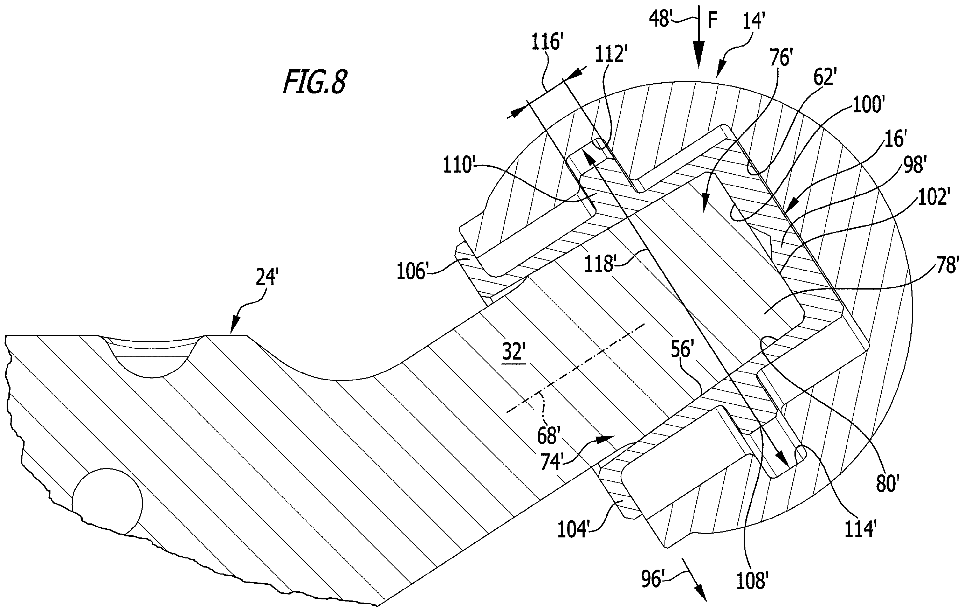

Das Kopplungselement 16' greift in das Sackloch 56' des Probegelenkelements 14' ein, sodass die Stirnwand 98' am Boden 62' des Sacklochs 56' anliegt. In dieser, in den Figuren 7 und 8 schematisch dargestellten Stellung, greift der Ringflansch 108' in die Ringnut 114' ein. Ein minimaler Innendurchmesser 118' der Ringnut 114' ist größer als ein maximaler Außendurchmesser 120' des Ringflanschs 108'. Eine Höhe 122' des Ringflanschs 108' in radialer Richtung ist etwas größer als eine halbe Tiefe 126' der Ringnut 114' in radialer Richtung.

Das Kopplungselement 16' und das Probegelenkelement 14' sind axial bezogen auf die Kopplungselementlängsachse 68' relativ zueinander festgelegt. Die in distaler Richtung weisende Endfläche 86' des Probegelenkelements 14' bildet im Zusammenwirken mit dem Ring 106' eine Messeinrichtung 50' sowie eine Anzeigeeinrichtung 52' zum Messen der Kraft 48' die auf das Probegelenkelement 14' wirkt. Die Endfläche 86' bildet ein Anzeigeelement 82', der Ring 106' eine Referenzelement 84'. Der Ring 106' weist einen Außendurchmesser 130' auf, der kleiner ist als ein Außendurchmesser 128' der Endfläche 86'. Damit ist unabhängig von einer Relativstellung zwischen dem Probegelenkelement 14' und dem Kopplungselement 16' stets ein Teil der Endfläche 86' sichtbar.

In einer Grundstellung des Messinstruments 12', wie sie schematisch in Figur 7 dargestellt ist, sind sowohl die Endfläche 86' als auch der Ring 106' koaxial zur Kopplungselementlängsachse 68' ausgerichtet.

Wirkt eine Kraft 48' auf das Probegelenkelement 14', wird dieses in einer Ebene quer zur Kopplungselementlängsachse 68' relativ zum Kopplungselement 16 verschoben. Dadurch wird die in der Grundstellung in Form eines Kreisrings sichtbare Endfläche 86' quasi unrund verformt, da der Ring 106' die Endfläche 86' auf der der Kraft 48' zugewandten Seite weiter überdeckt. Auf der gegenüberliegenden Seite wird die Endfläche 86' folglich mehr sichtbar.

Beispielsweise kann die Endfläche 86' in einer anderen Farbe eingefärbt werden als die Probegelenkfläche 36', sodass sich das Anzeigeelement 82' noch deutlicher vom Referenzelement 84' abhebt und für einen Operateur erkennbar ist. Durch die Verschiebung des Probegelenkelements 14' und des Kopplungselements 16' relativ zueinander kann ein Operateur direkt anhand der Verformung des sichtbaren Teils der Endfläche 86' eine Größe und/oder Orientierung der Kraft 48' erkennen.

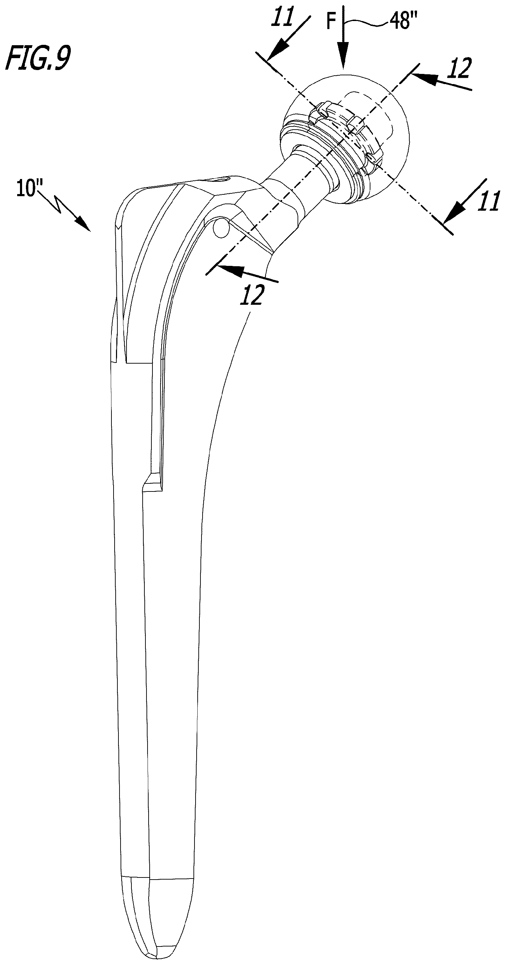

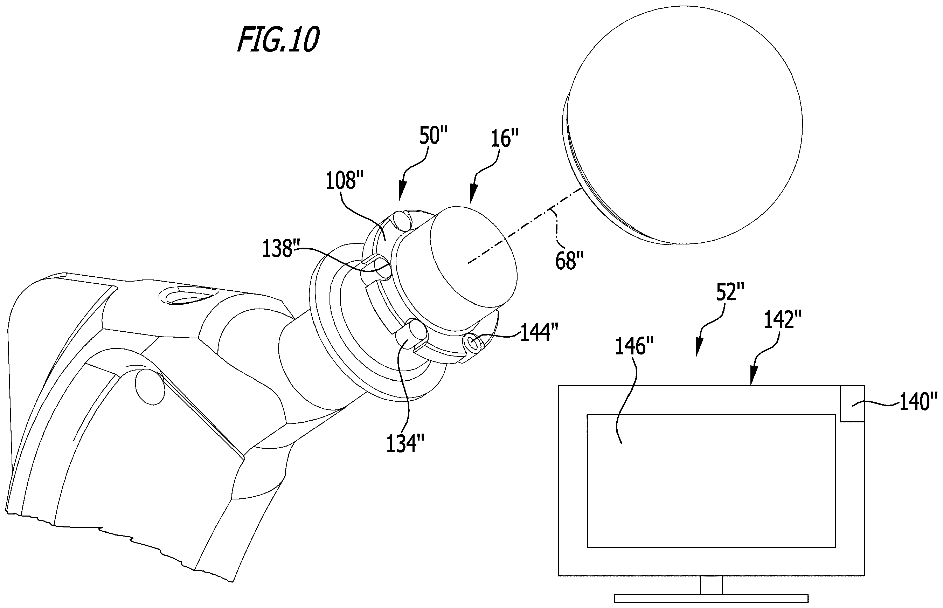

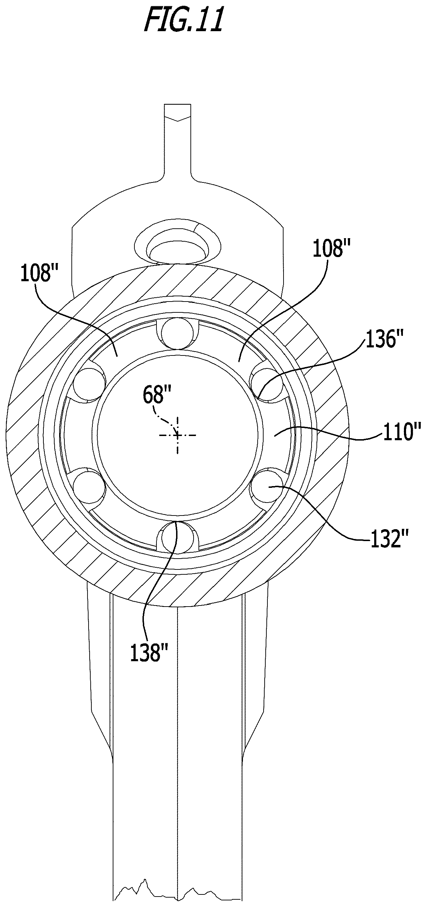

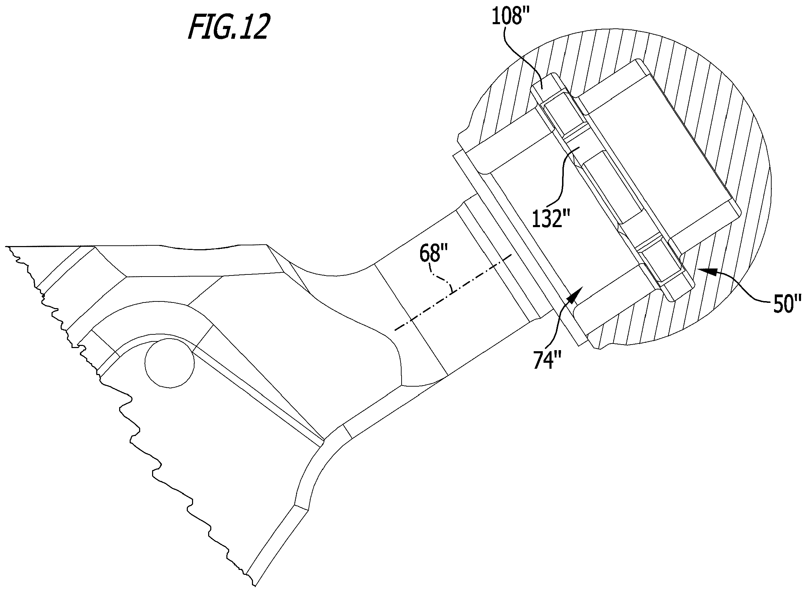

In den Figuren 9 bis 12 ist ein drittes Ausführungsbeispiel eines insgesamt mit dem Bezugszeichen 10" bezeichneten Kraftmesssystems beispielhaft dargestellt. Es stimmt in seinem grundsätzlichen Aufbau mit dem Kraftmesssystem 10' überein und unterscheidet sich lediglich in der Ausgestaltung des Kopplungselement 16" vom Kopplungselement 16' des Kraftmesssystems 10'. Zur Vermeidung von Wiederholungen wird daher betreffend den Aufbau und die Ausgestaltung des grundsätzlichen Aufbaus des Kraftmesssystems 10" auf die obigen Ausführungen zum Kraftmesssystem 10' verwiesen.

Das Kopplungselement 16" umfasst mehrere Kraftmesssensoren 132", nämlich genau sechs, die in Form kleiner Zylinder 134" ausgebildet und in Sensoraufnahmen 136" angeordnet sind. Die Sensoraufnahmen 136" sind in Form von halbzylindrischen Ausnehmungen 138" am Ringflansch 108" ausgebildet. Ein von den Ausnehmungen 138" definierter Innendurchmesser ist größer als ein Außendurchmesser der Kraftmesssensoren 132". Die Ausnehmungen 138" sind von der Kopplungselementlängsachse 68" weg weisend in Richtung auf das Probegelenkelement 14' geöffnet. Die Kraftmesssensoren 132" sind somit am Kopplungsglied 110" des Kopplungselements 16" angeordnet beziehungsweise gehalten.

Die Messeinrichtung 50" umfasst somit die Mehrzahl von Kraftmesssensoren 132" zum Messen einer Größe und/oder Richtung einer auf das Probegelenkelement 14' wirkenden Kraft 48".

Die Kraftmesssensoren 132" können insbesondere in Form elektronischer Kraftmesssensoren 132" ausgebildet sein zum Erzeugen eines elektrischen Messsignals. Zum Übertragen des Messsignals an eine Empfangseinrichtung 140" einer Signalanzeigeeinrichtung 142" kann der Kraftmesssensor 132" eine Sendeeinrichtung 144" zum Senden des Messsignals an die Empfangseinrichtung 140" umfassen. Ferner kann die Signalanzeigeeinrichtung 142" zum Auswerten und/oder Anzeigen des Messsignals ausgebildet sein. Die Signalanzeigeeinrichtung 142" kann insbesondere einen Bildschirm 146" umfassen. Eine Übertragung der Messsignale von der Sendeeinrichtung 144" zur Empfangseinrichtung 140" kann kabelgebunden oder kabellos erfolgen. Eine kabellose Übertragung kann insbesondere mittels Bluetooth oder Funk erfolgen.

Wirkt die Kraft 48" auf das Probegelenkelement 14', so kann eine Richtung und/oder Orientierung der Kraft 48" mittels der Messeinrichtung 50" und deren Kraftmesssensoren 132" ermittelt werden. Die Auswertung der Messsignale der Kraftmesssensoren 132" kann beispielsweise auf dem Bildschirm 146" für einen Operateur dargestellt werden, sodass dieser weiß, wie er gegebenenfalls eine Weichteilsituation ändern muss, um ein Kräfteungleichgewicht an der Gelenkprothese zu vermeiden.

### Bezugszeichenliste

- 10, 10', 10": Kraftmesssystem
- 12,12',12": Messinstrument
- 14, 14': Probegelenkelement
- 16, 16', 16": Kopplungselement
- 18: Gelenkprothese
- 20: Kugelgelenk
- 22: Teilkomponente
- 24, 24': Prothesenteil
- 26: Prothesenteil
- 28: Hüftgelenkendoprothese
- 30: Schaft
- 32, 32': Hals
- 34: Probegelenkkopf
- 36, 36': Probegelenkfläche
- 38: Gelenkfläche
- 40: Hüftpfanne
- 42: Pfanneneinsatz
- 44: Gelenkelement
- 46: Probekugelgelenk
- 48, 48', 48": Kraft
- 50, 50', 50": Messeinrichtung
- 52, 52": Anzeigeeinrichtung
- 54: Ausnehmung
- 56, 56': Sackloch
- 58: Ring
- 60, 60': Endfläche
- 62, 62': Boden
- 64: Ringnut
- 66: Klemmelement
- 68, 68', 68": Kopplungselementlängsachse
- 70: Ringnut
- 72: Klemmring
- 74, 74', 74": Messinstrumentenkopplungseinrichtung
- 76, 76': Kopplungseinrichtung
- 78, 78': Kopplungsvorsprung
- 80, 80': Kopplungsaufnahme
- 82, 82': Anzeigeelement
- 84, 84': Referenzelement
- 86, 86': Endfläche
- 88: Lamelle
- 90: Streifen
- 92: Anlageabschnitt
- 94: Ende
- 96: Richtung
- 98': Stirnwand
- 100': Innenseite
- 102': Endfläche
- 104: Ringflansch
- 106': Ring
- 108': Ringflansch
- 110': Kopplungsglied
- 112': Kopplungsglied
- 114': Ringnut
- 116': Breite
- 118': Innendurchmesser
- 120': Außendurchmesser
- 122': Höhe
- 126': Tiefe
- 128': Außendurchmesser
- 130': Außendurchmesser
- 132": Kraftmesssensor
- 134": Zylinder
- 136": Sensoraufnahme
- 138": Ausnehmung
- 140": Empfangseinrichtung
- 142": Signalanzeigeeinrichtung
- 144": Sendeeinrichtung
- 146": Bildschirm

## Patentansprüche

1. Medizinisches Kraftmesssystem (10; 10'; 10") zum Messen einer in einer Gelenkprothese (18; 18') umfassend ein Kugelgelenk (20) oder einer Teilkomponente (22) derselben zwischen zwei gelenkig miteinander verbundenen Prothesenteilen (24, 26; 24') wirkenden Kraft (48; 48'; 48"), welches Kraftmesssystem (10; 10'; 10") ein Messinstrument (12; 12'; 12") umfasst, welches Messinstrument (12; 12'; 12") ein Probegelenkelement (14; 14') mit einer Probegelenkfläche (36; 36') umfasst, welches Probegelenkelement (14; 14') korrespondierend zu einem ersten, einen Teil des Kugelgelenks (20) bildenden und eine korrespondierend zur Probegelenkfläche (36; 36') ausgebildete Gelenkfläche (38) aufweisenden Gelenkelement (44) ausgebildet und mit diesem in Eingriff bringbar ist zum Ausbilden eines Probekugelgelenks (46), **dadurch gekennzeichnet, dass** das Messinstrument (12; 12'; 12") eine an dem Probegelenkelement (14, 14') angeordnete Anzeigeeinrichtung (52; 52'; 52") umfasst zum Anzeigen einer auf die Probegelenkfläche (36; 36') wirkenden Kraft oder einer Teilkomponente der auf die Probegelenkfläche (36; 36') wirkenden Kraft (48; 48'; 48").

2. Medizinisches Kraftmesssystem nach Anspruch 1, **dadurch gekennzeichnet, dass** das Messinstrument (12; 12'; 12") eine Messinstrumentenkopplungseinrichtung (74; 74'; 74") umfasst zum temporären, kraft- und/oder formschlüssigen Koppeln mit einer Kopplungseinrichtung (76; 76') eines Prothesenteils (24; 24') der Gelenkprothese (18) oder einem medizinischen Instrument
und/oder
dass das medizinische Kraftmesssystem ein medizinisches Instrument umfasst, welches das Messinstrument (12; 12'; 12") umfasst oder mit dem Messinstrument (12; 12'; 12") koppelbar ist.

3. Medizinisches Kraftmesssystem nach Anspruch 2, **dadurch gekennzeichnet, dass** das medizinische Instrument in Form einer Raspel oder eines Raspelschafts ausgebildet ist
und/oder
dass das medizinische Instrument eine Instrumentenkopplungseinrichtung (76; 76') umfasst, welche mit der Messinstrumentenkopplungseinrichtung (74; 74'; 74") kraft- und/oder formschlüssig in Eingriff bringbar ist
und/oder
dass die Instrumentenkopplungseinrichtung (76; 76') oder die Messinstrumentenkopplungseinrichtung (74; 74'; 74") in Form eines Kopplungsvorsprungs (78; 78') ausgebildet ist und dass die jeweils andere Kopplungseinrichtung (74; 74'; 74") in Form einer zum Kopplungsvorsprung (78; 78') korrespondierenden Kopplungsaufnahme (80; 80') ausgebildet ist.

4. Medizinisches Kraftmesssystem nach einem der voranstehenden Ansprüche, **gekennzeichnet durch** ein Prothesenteil (24; 24') der Gelenkprothese (18), welches mit dem Messinstrument (12; 12'; 12") temporär koppelbar ist.

5. Medizinisches Kraftmesssystem nach einem der voranstehenden Ansprüche, **dadurch gekennzeichnet, dass** das Probegelenkelement (14; 14') in Form eines Probegelenkkopfs (34) oder in Form eines Pfanneneinsatzes (42) einer Hüftgelenkprothese (28) ausgebildet ist.

6. Medizinisches Kraftmesssystem nach einem der voranstehenden Ansprüche, **dadurch gekennzeichnet, dass** das Messinstrument (12; 12'; 12") ein Kopplungselement (16; 16'; 16") umfasst und dass das Kopplungselement (16; 16'; 16") die Messinstrumentenkopplungseinrichtung (74; 74'; 74") umfasst.

7. Medizinisches Kraftmesssystem nach Anspruch 6, **dadurch gekennzeichnet, dass** das Probegelenkelement (14; 14') und das Kopplungselement (16; 16'; 16") relativ zueinander beweglich angeordnet oder aneinander gelagert oder relativ zueinander beweglich ausgebildet sind
und/oder
dass das Probegelenkelement (14; 14') und das Kopplungselement (16; 16'; 16") relativ zueinander verschiebbar und/oder verdrehbar angeordnet oder ausgebildet sind
und/oder
dass die Instrumentenkopplungseinrichtung ein Klemmelement (66) umfasst, welches einerseits mit dem Prothesenteil (24) oder dem medizinischen Instrument und andererseits mit dem Kopplungselement (16) kraft- und/oder formschlüssig in Eingriff bringbar ist.

8. Medizinisches Kraftmesssystem nach einem der voranstehenden Ansprüche, **dadurch gekennzeichnet, dass** die Anzeigeeinrichtung (52) mindestens ein Anzeigeelement (82) und mindestens ein Referenzelement (84) umfasst und dass das mindestens eine Anzeigeelement (82) und das mindestens eine Referenzelement (84) relativ zueinander beweglich angeordnet oder ausgebildet sind.

9. Medizinisches Kraftmesssystem nach Anspruch 8, **dadurch gekennzeichnet, dass** die Anzeigeeinrichtung (52) eine Mehrzahl von Anzeigeelementen (82) umfasst, welche in Folge einer Krafteinwirkung auf das Probegelenkelement und/oder das Kopplungselement (16) aus einer Grundstellung auslenkbar und/oder temporär verformbar sind.

10. Medizinisches Kraftmesssystem nach Anspruch 9, **dadurch gekennzeichnet, dass** die Mehrzahl von Anzeigeelementen (82) die Kopplungsaufnahme (80) in Umfangsrichtung umgebend angeordnet oder ausgebildet ist.

11. Medizinisches Kraftmesssystem nach einem der Ansprüche 8 bis 10, **dadurch gekennzeichnet, dass** das mindestens eine Anzeigeelement (82') in Form einer Ringfläche (86') am Probegelenkelement (14') oder am Kopplungselement ausgebildet ist und dass das mindestens eine Referenzelement (84') in Form eines Rings (106') ausgebildet ist, welcher einen kleineren Durchmesser (128') aufweist als die Ringfläche (86') und diese mindestens teilweise überdeckt.

12. Medizinisches Kraftmesssystem nach Anspruch 11, **dadurch gekennzeichnet, dass** die Ringfläche (86') in Form einer ebenen, vom Probegelenkelement (14') weg weisenden Endfläche (86') ausgebildet ist, welche eine Ausnehmung (54') des Probegelenkelements (14') umgibt, in welche das Kopplungselement (16') eingreift, und dass das Kopplungselement (16') einen die Kopplungsaufnahme (80') umgebenden, von einer Kopplungsaufnahmelängsachse (68') radial weg weisenden Ringflansch (104') umfasst, welcher den Ring (106') bildet.

13. Medizinisches Kraftmesssystem nach einem der voranstehenden Ansprüche, **dadurch gekennzeichnet, dass** das Messinstrument (12; 12'; 12") eine mit dem Probegelenkelement (14; 14') zusammenwirkende Messeinrichtung (50; 50'; 50") umfasst zum Messen einer auf die Probegelenkfläche (36; 36') wirkenden Kraft oder einer Teilkomponente der auf die Probegelenkfläche (36; 36') wirkenden Kraft (48; 48'; 48"),
wobei insbesondere
die Messeinrichtung (50") mindestens einen Kraftmesssensor (132") umfasst zum Messen einer Größe und/oder Richtung einer auf das Probegelenkelement (14') wirkenden Kraft.

14. Medizinisches Kraftmesssystem nach Anspruch 13, **dadurch gekennzeichnet, dass** der mindestens eine Kraftmesssensor (132") an einem der zusammenwirkenden Kopplungsglieder (110") angeordnet ist und/oder dass mindestens eines der Kopplungsglieder (110") mindestens eine Sensoraufnahme (136") zum Aufnehmen des mindestens einen Kraftmesssensors (132") umfasst
und/oder
dass der mindestens eine Kraftmesssensor (132") in Form eines elektronischen Kraftmesssensors (132") ausgebildet ist zum Erzeugen eines elektrischen Messsignals
und/oder
dass der mindestens eine Kraftmesssensor (132") eine Sendeeinrichtung (144") umfasst zum Senden eines Messsignals an eine Empfangseinrichtung (140") einer Signalanzeigeeinrichtung (142") zum Auswerten und/oder Anzeigen des Messsignals.

15. Medizinisches Kraftmesssystem nach einem der voranstehenden Ansprüche, **dadurch gekennzeichnet, dass** das Probegelenkelement (14; 14') einstückig ausgebildet ist und/oder dass das Kopplungselement (16; 16'; 16") einstückig ausgebildet ist.

## Claims

1. Medical force measuring system (10; 10'; 10") for measuring a force (48; 48'; 48") that is effective between two prosthesis parts (24, 26; 24') that are connected to one another in articulated manner in a joint prosthesis (18; 18') comprising a ball joint (20) or a partial component (22) thereof, which force measuring system (10; 10'; 10") comprises a measuring instrument (12; 12'; 12"), which measuring instrument (12; 12'; 12") comprises a sample joint element (14; 14') having a sample joint surface (36; 36'), which sample joint element (14; 14') is formed in correspondence with a first joint element (44) that forms a part of the ball joint (20) and comprises a joint surface (38) that is formed in correspondence with the sample joint surface (36; 36') and is moveable into engagement therewith for forming a sample ball joint (46), **characterized in that** the measuring instrument (12; 12'; 12") comprises an indicating device (52; 52'; 52") which is arranged on the sample joint element (14; 14') for indicating a force that is effective on the sample joint surface (36; 36') or a partial component of the force (48; 48'; 48") that is effective on the sample joint surface (36; 36').

2. Medical force measuring system in accordance with Claim 1, **characterized in that** the measuring instrument (12; 12'; 12") comprises a measuring instrument coupling device (74; 74'; 74") for temporarily coupling in force- and/or positive-locking manner to a coupling device (76; 76') of a prosthesis part (24; 24') of the joint prosthesis (18) or a medical instrument,
and/or
**in that** the medical force measuring system comprises a medical instrument which comprises the measuring instrument (12; 12'; 12") or is couplable to the measuring instrument (12; 12'; 12").

3. Medical force measuring system in accordance with Claim 2, **characterized in that** the medical instrument is in the form of a rasp or a rasp shaft,
and/or
**in that** the medical instrument comprises an instrument coupling device (76; 76') which is moveable into engagement with the measuring instrument coupling device (74; 74'; 74") in force-and/or positive-locking manner,
and/or
**in that** the instrument coupling device (76; 76') or the measuring instrument coupling device (74; 74'; 74") is in the form of a coupling projection (78; 78') and **in that** the respective other coupling device (74; 74'; 74") is in the form of a coupling seating (80; 80') corresponding to the coupling projection (78; 78').

4. Medical force measuring system in accordance with any one of the preceding Claims, **characterized by** a prosthesis part (24; 24') of the joint prosthesis (18) which is couplable temporarily to the measuring instrument (12; 12'; 12").

5. Medical force measuring system in accordance with any one of the preceding Claims, **characterized in that** the sample joint element (14; 14') is in the form of a sample joint head (34) or in the form of a socket insert (42) of a hip joint prosthesis (28).

6. Medical force measuring system in accordance with any one of the preceding Claims, **characterized in that** the measuring instrument (12; 12'; 12") comprises a coupling element (16; 16'; 16") and **in that** the coupling element (16; 16'; 16") comprises the measuring instrument coupling device (74; 74'; 74").

7. Medical force measuring system in accordance with Claim 6, **characterized in that** the sample joint element (14; 14') and the coupling element (16; 16'; 16") are arranged to be moveable relative to each other or are mounted on one another or are formed such as to be moveable relative to each other,
and/or
**in that** the sample joint element (14; 14') and the coupling element (16; 16'; 16") are arranged or formed to be displaceable and/or rotatable relative to each other,
and/or
**in that** the instrument coupling device comprises a clamping element (66) which is moveable into engagement in force- and/or positive-locking manner with the prosthesis part (24) or the medical instrument on the one hand and with the coupling element (16) on the other.

8. Medical force measuring system in accordance with any one of the preceding Claims, **characterized in that** the indicating device (52) comprises at least one indicating element (82) and at least one reference element (84) and **in that** the at least one indicating element (82) and the at least one reference element (84) are arranged or formed such as to be moveable relative to each other.

9. Medical force measuring system in accordance with Claim 8, **characterized in that** the indicating device (52) comprises a plurality of indicating elements (82) which are deflectable from a basic position and/or are temporarily deformable as a result of an application of force to the sample joint element and/or the coupling element (16).

10. Medical force measuring system in accordance with Claim 9, **characterized in that** the plurality of indicating elements (82) is arranged or formed around the coupling seating (80) in the circumferential direction.

11. Medical force measuring system in accordance with any one of the Claims 8 to 10, **characterized in that** the at least one indicating element (82') is in the form of an annular surface (86') on the sample joint element (14') or on the coupling element, and **in that** the at least one reference element (84') is in the form of a ring (106') which is of smaller diameter (128') than the annular surface (86') and at least partly covers it.

12. Medical force measuring system in accordance with Claim 11, **characterized in that** the annular surface (86') is in the form of a flat end face (86') which faces away from the sample joint element (14') and surrounds a recess (54') of the sample joint element (14') with which the coupling element (16') engages, and **in that** the coupling element (16') comprises a ring flange (104') which surrounds the coupling seating (80') and is directed radially away from a longitudinal axis (68') of the coupling seating and also forms the ring (106').

13. Medical force measuring system in accordance with any one of the preceding Claims, **characterized in that** the measuring instrument (12; 12'; 12") comprises a measuring device (50; 50'; 50") which cooperates with the sample joint element (14; 14') for measuring a force that is effective on the sample joint surface (36; 36') or a partial component of the force (48; 48';48") that is effective on the sample joint surface (36; 36'),
wherein, in particular,
the measuring device (50") comprises at least one force measuring sensor (132") for measuring the magnitude and/or direction of a force effective on the sample joint element (14').

14. Medical force measuring system in accordance with Claim 13, **characterized in that** the at least one force measuring sensor (132") is arranged on one of the cooperating coupling members (110") and/or **in that** at least one of the coupling members (110") comprises at least one sensor seating (136") for accommodating the at least one force measuring sensor (132"),
and/or
**in that** the at least one force measuring sensor (132") is in the form of an electronic force measuring sensor (132") for producing an electrical measuring signal,
and/or
**in that** the at least one force measuring sensor (132") comprises a transmitting device (144") for sending a measuring signal to a receiving device (140") of a signal indicator device (142") for evaluating and/or indicating the measuring signal.

15. Medical force measuring system in accordance with any one of the preceding Claims, **characterized in that** the sample joint element (14; 14') is formed in one piece manner and/or **in that** the coupling element (16; 16'; 16") is formed in one piece manner.

## Revendications

1. Système médical de mesure de force (10; 10'; 10") pour mesurer une force (48; 48'; 48") agissant dans une prothèse d'articulation (18; 18') comprenant une articulation à rotule (20), ou dans un composant partiel (22) de celle-ci, entre deux parties de prothèse (24, 26; 24') reliées l'une à l'autre de manière articulée, ledit système de mesure de force (10; 10'; 10") comprenant un instrument de mesure (12; 12'; 12"), ledit instrument de mesure (12; 12'; 12") comprenant un élément d'articulation d'essai (14; 14') avec une surface d'articulation d'essai (36; 36'), et ledit élément d'articulation d'essai (14; 14') étant réalisé de manière correspondante à un premier élément d'articulation (44), qui forme une partie de l'articulation à rotule (20) et présente une surface d'articulation (38) réalisée de manière correspondante à la surface d'articulation d'essai (36; 36'), et pouvant être amené en prise avec ce premier élément d'articulation pour former une articulation à rotule d'essai (46),
**caractérisé en ce que** l'instrument de mesure (12; 12'; 12") comporte un dispositif indicateur (52; 52'; 52") agencé sur l'élément d'articulation d'essai (14; 14'), pour indiquer une force agissant sur la surface d'articulation d'essai (36; 36') ou une composante partielle de la force (48; 48'; 48") agissant sur la surface d'articulation d'essai (36; 36').

2. Système médical de mesure de force selon la revendication 1, **caractérisé en ce que** l'instrument de mesure (12; 12'; 12") comprend un dispositif de couplage d'instrument de mesure (74; 74'; 74") pour assurer le couplage temporaire par adhérence et/ou complémentarité de formes avec un dispositif de couplage (76; 76") d'une pièce de prothèse (24; 24') de la prothèse d'articulation (18) ou d'un instrument médical, et/ou
**en ce que** le système médical de mesure de force comprend un instrument médical, qui comprend l'instrument de mesure (12; 12'; 12") ou peut être couplé à l'instrument de mesure (12; 12'; 12").

3. Système médical de mesure de force selon la revendication 2, **caractérisé en ce que** l'instrument médical est réalisé sous la forme d'une râpe ou d'un corps de râpe,
et/ou
**en ce que** l'instrument médical comprend un dispositif de couplage d'instrument (76; 76'), qui peut être amené en prise par adhérence et/ou complémentarité de formes, avec le dispositif de couplage d'instrument de mesure (74; 74'; 74"),
et/ou
**en ce que** le dispositif de couplage d'instrument (76; 76') ou le dispositif de couplage d'instrument de mesure (74; 74"; 74") est réalisé sous la forme d'une protubérance de couplage (78; 78'), et **en ce que** respectivement l'autre dispositif de couplage (74; 74'; 74") est réalisé sous la forme d'un logement de couplage (80; 80') correspondant à la protubérance de couplage (78; 78').

4. Système médical de mesure de force selon l'une des revendications précédentes, **caractérisé par** une partie de prothèse (24; 24') de la prothèse d'articulation (18), qui peut être couplée temporairement à l'instrument de mesure (12; 12'; 12").

5. Système médical de mesure de force selon l'une des revendications précédentes, **caractérisé en ce que** l'élément d'articulation d'essai (14; 14') est réalisé sous la forme d'une tête d'articulation d'essai (34) ou sous la forme d'un insert de cupule (42) d'une prothèse d'articulation de la hanche (28).

6. Système médical de mesure de force selon l'une des revendications précédentes, **caractérisé en ce que** l'instrument de mesure (12; 12'; 12") comprend un élément de couplage (16; 16'; 16"), et **en ce que** l'élément de couplage (16; 16'; 16") comporte le dispositif de couplage d'instrument de mesure (74; 74'; 74").

7. Système médical de mesure de force selon la revendication 6, **caractérisé en ce que** l'élément d'articulation d'essai (14; 14') et l'élément de couplage (16; 16'; 16") sont agencés ou montés l'un contre l'autre de manière mobile l'un par rapport à l'autre, ou bien réalisés mobiles l'un par rapport à l'autre,
et/ou
**en ce que** l'élément d'articulation d'essai (14; 14') et l'élément de couplage (16; 16'; 16") sont agencés ou réalisés de manière à pouvoir coulisser et/ou tourner l'un par rapport à l'autre,
et/ou
**en ce que** le dispositif de couplage d'instrument comporte un élément d'arrêt (66), qui peut être amené en prise par adhérence et/ou complémentarité de formes, d'une part avec la partie de prothèse (24) ou l'instrument médical, et d'autre part avec l'élément de couplage (16).

8. Système médical de mesure de force selon l'une des revendications précédentes, **caractérisé en ce que** le dispositif indicateur (52) comprend au moins un élément indicateur (82) et au moins un élément de référence (84), et **en ce que** ledit au moins un élément indicateur (82) et ledit au moins un élément de référence (84) sont agencés ou réalisés de manière à être mobiles l'un par rapport à l'autre.

9. Système médical de mesure de force selon la revendication 8, **caractérisé en ce que** le dispositif indicateur (52) comprend une pluralité d'éléments indicateurs (82), qui, suite à l'action d'une force sur l'élément d'articulation d'essai et/ou l'élément de couplage (16), peuvent être déviés d'une position de base et/ou être déformés temporairement.

10. Système médical de mesure de force selon la revendication 9, **caractérisé en ce que** la pluralité d'éléments indicateurs (82) sont agencés ou réalisés de manière à entourer, dans la direction périphérique, le logement de couplage (80).

11. Système médical de mesure de force selon l'une des revendications 8 à 10, **caractérisé en ce que** ledit au moins un élément indicateur (82') est réalisé sous la forme d'une surface annulaire (86') sur l'élément d'articulation d'essai (14') ou sur l'élément de couplage, et **en ce que** ledit au moins un élément de référence (84') est réalisé sous la forme d'un anneau (106'), qui présente un diamètre (128') inférieur à celui de la surface annulaire (86') et recouvre celle-ci au moins partiellement.

12. Système médical de mesure de force selon la revendication 11, **caractérisé en ce que** la surface annulaire (86') est réalisée sous la forme d'une surface d'extrémité (86') plane, orientée dans une direction s'éloignant de l'élément d'articulation d'essai (14'), et qui entoure un évidement (54') de l'élément d'articulation d'essai (14') dans lequel s'engage l'élément de couplage (16'), et **en ce que** l'élément de couplage (16') comporte un flasque annulaire (104'), qui entoure le logement de couplage (80'), s'éloigne radialement d'un axe longitudinal de logement de couplage (68'), et forme l'anneau (106').

13. Système médical de mesure de force selon l'une des revendications précédentes, **caractérisé en ce que** l'instrument de mesure (12; 12'; 12") comprend un dispositif de mesure (50; 50'; 50") interagissant avec l'élément d'articulation d'essai (14; 14') et destiné à mesurer une force agissant sur la surface d'articulation d'essai (36; 36') ou une composante partielle de la force (48; 48'; 48") agissant sur la surface d'articulation d'essai (36; 36'),
le dispositif de mesure (50") comprenant notamment un capteur de mesure de force (132") pour mesurer une grandeur et/ou une direction d'une force agissant sur l'élément d'articulation d'essai (14').

14. Système médical de mesure de force selon la revendication 13, **caractérisé en ce que** ledit au moins un capteur de mesure de force (132") est agencé sur l'un des organes de couplage (110") interagissant mutuellement, et/ou **en ce que** l'un au moins des organes de couplage (110") comporte un logement d'accueil de capteur (136") destiné à accueillir ledit au moins un capteur de mesure de force (132"),
et/ou
**en ce que** ledit au moins un capteur de mesure de force (132") est réalisé sous la forme d'un capteur de mesure de force (132") électronique, destiné à produire un signal de mesure électrique,
et/ou
**en ce que** ledit au moins un capteur de mesure de force (132") comporte un dispositif émetteur (144") pour envoyer un signal de mesure à un dispositif récepteur (140") d'un dispositif de visualisation de signal (142") destiné à exploiter et/ou visualiser le signal de mesure.

15. Système médical de mesure de force selon l'une des revendications précédentes, **caractérisé en ce que** l'élément d'articulation d'essai (14; 14') est réalisé d'un seul tenant, et/ou **en ce que** l'élément de couplage (16; 16'; 16") est réalisé d'un seul tenant.
